# EUROPEAN PATENT SPECIFICATION

(11) **EP 0 677 056 B1**
(45) Date of publication and mention of the grant of the patent: **22.05.1996**
(21) Application number: 94908639.1
(22) Date of filing: 25.01.1994
(51) Int. Cl.: C07H 21/00, C12N 15/11, A61K 31/70

(54) **OLIGONUCLEOTIDE ALKYLPHOSPHONATES AND ALKYLPHOSPHONOTHIOATES**
OLIONUKLEOTIDALKYLPHOSPHONATE UND -PHOSPHONOTHIOATE
ALKYLPHOSPHONATES ET ALKYLPHOSPHONOTHIOATES D'OLIGONUCLEOTIDES

(30) Priority: 25.01.1993 US 9262
(43) Date of publication of application: 18.10.1995
(73) Proprietor: HYBRIDON, Inc., Worcester, Massachusetts 01605 (US)
(72) Inventor: KANDIMALLA, Ekambar R., Worcester, MA 01604 (US); TEMSAMANI, Jamal, Shrewsbury, MA 01545 (US); AGRAWAL, Sudhir, Shrewsbury, MA 01545 (US)
(74) Representative: Vossius, Volker, Dr.
(86) International application number: US9400902
(87) International publication number: WO9417093

(56) References cited:
- EP-A- 0 136 543
- EP-A- 0 386 987
- EP-A- 0 552 766
- WO-A-91/06556
- WO-A-91/16902
- WO-A-92/04358
- WO-A-92/20967
- WO-A-94/02499
- US-A- 4 469 863
- TETRAHEDRON LETTERS. vol. 32, no. 37 , 9 September 1991 , OXFORD GB pages 4981 - 4984 J.HELINSKI ET AL. 'N,N-Diisopropyl-O-p-nit rophenyl-P-methylphosphonoamidate: Novel Difunctional Piii Reagent in Oligonucleoside Methylphosphonate Synthesis Containing 4-Nitrophenoxy Group.'
- TETRAHEDRON LETTERS. vol. 33, no. 17 , 21 April 1992 , OXFORD GB pages 2357 - 2360 H.C.P.F.ROELEN ET AL. 'Synthesis of Alkylphosphon(othio)ate Analogues of DNA.'

## Description

### FIELD OF THE INVENTION

This invention relates to synthetic oligonucleotides useful for antisense applications. More particularly, this invention relates to synthetic oligonucleotides having modifications in their sugar phosphate backbone which enhance their antisense qualities, and to uses thereof.

### BACKGROUND OF THE INVENTION

The sequence specific recognition and binding of various biological molecules to cellular nucleic acid targets is fundamental in molecular biological processes such as gene expression and control. Design of peptide based sequence specific agents for various uses including therapeutic purposes is very difficult as the sequence specific recognition and interaction between proteins and DNA is not very well understood to date. However, it is possible to design and develop sequence-specific oligonucleotides to bind any predetermined sequence on DNA through triple helix formation and on RNA through double helix formation because of the complementary recognition of nucleotide bases via hydrogen bonding interactions. Based on this principle, natural and modified oligonucleotides are used extensively as tools in molecular and cellular biology to determine the role played by specific genes in living systems.

In recent years oligonucleotides have been used to block expression of specific genes by interacting with mRNA species in cell culture systems (Uhlmann at al., (1990) *Chem. Rev.* 90: 543-584; Helene et al., (1990) *Biochem. Biophys. Acta* 1049:99-125). These "antisense" oligonucleotides can be used as therapeutic agents *in vivo.* For example, antisense oligonucleotides can be used to suppress smooth muscle cell migration or growth in carotid arteries of rats (Simons et al., (1992) *Nature* 359:67-70).

In order to be useful as therapeutic agents, antisense oligonucleotides must be able to be taken up by cells and function therein. Native phosphodiester-linked oligonucleotides are not taken up by cells efficiently because of their polyanionic nature. They are also highly susceptible to nuclease action within the cell (see, e.g., Wickstrom, *(1986) Biochem. Biophys. Meth.* 13:97-102), limiting their bioavailability in vivo. To improve cellular uptake and resistance to nuclease action, the sugar phosphate backbone of these oligonucleotides have been modified. For example, oligodeoxyribonucleotides containing methylphosphonate linkages have been prepared by Ts'o et al., (U.S. Patent No. 4,469,863) and Miller et al., (*Biochem.* (1981) 20:1874-1880). Other modifications include phosphorothioates, phosphorodithioates, phosphoramidates, and phosphate esters.

"Chimeric" oligodeoxyribonucleotides have been synthesized which have more than one type of internucleotide linkage, such as those modified linkages described above. For example, Pederson at al., (U.S. Patent No. 3,149,797) disclose chimeric oligonucleotides having an oligonucleotide phosphodiester or oligonucleotide phosphorothioate core sequence flanked by oligonucleotide methylphosphonates or phosphoramidates. Furdon at al. (*Nucleic Acids Res.* (1989) 17:9193-9204) disclose chimeric oligodeoxyribonucleotides having regions of oligonucleotide phosphodiesters in addition to either oligodeoxyribonucleotide phosphorothioate or methylphosphonate regions. Quartin at al. (Nucleic Acids Res. (1989) 17:(7523-7562) disclose chimeric oligodeoxyribonucleotides having regions of oligonucleotide phosphodiester and oligonucleotide methylphosphonates.

Many of these modified oligonucleotides have contributed to improving the potential efficacy of the antisense oligonucleotide therapeutic approach. However, certain deficiencies remain that limit the effectiveness of such oligonucleotides as therapeutic agents. For example, Agrawal et al. (*Proc. Natl. Acad. Sci. (USA)* 87:1401-1405) teach that oligodeoxyribonucleotide phosphoramidates when hybridized to RNA do not activate RNase H, the activation of which can be important to the function of antisense oligonucleotides. Also, Agrawal et al. (*Nucleosides & Nucleotides* (1989) 8:5-6) teach that oligodeoxyribonucleotide phosphorothioates have reduced duplex stability when hybridized to RNA.

Thus, there is a need for improved oligonucleotides that overcome the deficiencies of oligonucleotides known in the art. Ideally, such oligonucleotides should be resistant to nucleolytic degradation and should form stable duplexes with RNA.

### SUMMARY OF THE INVENTION

In a first aspect, the invention provides synthetic oligonucleotide analogs that have been prepared with nonionic internucleotide linkages. The subject oligonucleotide analogs are highly nuclease resistant, form a very stable duplex with RNA, and have the requisite hydrophobicity for successful cellular uptake. A requirement of these analogs is that they have at least one ribonucleotide alkylphosphonate or alkylphosphonothioate at any selected position(s) within the oligonucleotide and/or at either or both ends.

For purposes of the invention, the term "oligonucleotide" includes polymers of one or more ribonucleotide monomers connected together or linked to a deoxyribonucleotide monomer by at least one 5' to 3' internucleotide linkage. These linkages include any of the linkages known in the antisense oligonucleotide art. In addition, the term "oligonucleotide" includes molecules having modified nucleic acid/bases and/or sugars, added substituents, such as diamines, cholesteryl or other lipophilic groups, and 2'-substituted ribonucleoside monomers. The term "oligonucleotide analog" encompasses oligonucleotides with at least one non-phosphodiester internucleotide linkage and/or with modified nucleic acid/bases and/or sugars.

In a preferred embodiment, oligonucleotide analogs according to the invention range from about 2 to about 60 nucleotides in length, and most preferably from about 25 to about 30 nucleotides in length. Thus, oligonucleotides according to the invention will have from 1 to about 59 ribonucleotide alkylphosphonates or alkylphosphonothioates, or a combination of such linkages, and preferably from about 24 to 29 groups.

As used herein, an alkylphosphonate or alkylphosphonothioate is a deoxyribonucleotide or ribonucleotide, or 2'-substituted ribonucleotide, having a phosphonate or phosphonothioate linkage with a 2' alkyl group, including but not limited to, methyl, ethyl, propyl, or butyl group.

In some embodiments of the invention, the oligonucleotide analogs have alkylphosphonate and/or alkylphosphonothioates at some positions within the molecule and natural nucleotide linked by phosphodiester and/or artificial linkages at other positions. These are considered "chimeric" or "hybrid" oligonucleotides.

As used herein, an artificial internucleotide linkage is a non-phosphodiester linkage which links ribonucleotide monomers together from 5' to 3', and which includes, but is not limited to, phosphorothioates, phosphorodithioates, carbonates, phosphate esters, phosphoramidates, and carbamates. In another embodiment of the invention, the oligonucleotide analogs include at least one, and preferably 6 to 10 2'-substituted ribonucleosides. For purposes of the invention, the term "2'-substituted" means substitution of the 2'-OH of the ribose molecule with, *e.g.,* 2'-O-methyl, 2'-allyl, 2'-aryl, 2'-alkyl, 2'-halo, or 2'-amino, but not with 2'-H, wherein allyl, aryl, or alkyl groups may be unsubstituted or substituted, *e.g.,* with halo, hydroxy, cyano, nitro, acyl, acyloxy, alkoxy, carboxyl, trifluoromethyl, carbalkoxyl, or amino groups.

In a second aspect, the invention provides therapeutic pharmaceutical formulations that are effective for treating viral infection, infections by pathogenic organisms, or disease resulting from abnormal gene expression or from the expression of an abnormal gene product. Such therapeutic pharmaceutical formulations include the oligonucleotides according to the first aspect of the invention in a physiologically acceptable carrier.

In a third aspect, the invention provides a method for inhibiting the expression of a viral gene, a gene of a pathogenic organism, or a cellular gene using the oligonucleotide analogs described above. The method includes treating the cells infected with the virus or pathogenic organism in the former two cases, or the cells, generally, in the latter case, with the oligoribonucleotide analog of the invention.

In a fourth aspect, the invention provides a method of treating a mammal afflicted with a disease or disorder resulting from infection with a virus or pathogenic organism, or resulting from the abnormal expression or product of a cellular gene. The method includes administering therapeutic pharmaceutical formulations including the oligonucleotide analogs according to the invention to the mammal in an amount sufficient to form a stable duplex with the nucleic acid of the virus, pathogen, or gene, thereby inactivating it. Preferable routes of such administration include oral, intranasal, rectal, intravenous, and topical administration. This method of treatment may also include the administration of other therapeutic agents in conjunction with the oligonucleotide analog-containing formulation.

### BRIEF DESCRIPTION OF THE DRAWINGS

The foregoing and other objects of the present invention, the various features thereof, as well as the invention itself may be more fully understood from the following description, when read together with the accompanying drawings in which:
FIG. 1 is a schematic representation of various phosphate backbone linkages and modifications: FIG. 1A, phosphodiester; FIG. 1B, methylphosphonothioate; FIG. 1C, methylphosphonate; FIG. 1D, phosphorothioate; FIG. IE, phosphorodithioate; 1F, phosphoramidate; and 1G, phosphate ester;
FIG. 2 is a schematic representation of the synthesis of 3'0-phosphanamidites and their subsequent incorporation into an oligonucleotide;

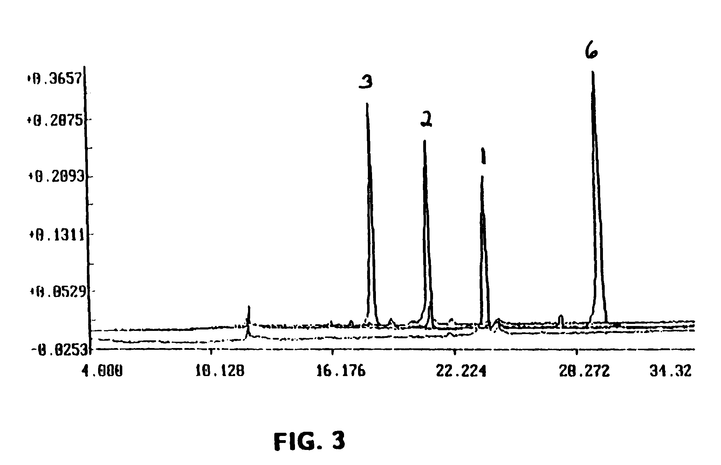

FIG. 3 is a graphic representation comparing ion exchange HPLC profiles of 11mer analogs 1 to 3 (SEQ ID NOS:1-3) and control d(A) 11mer;
FIG. 4 is a graphic representation of the melting characteristics of duplexes formed from the 25mer analog (SEQ ID NO:4) and complementary strands of (A) DNA and (B) RNA;
FIG. 5 is a graphic representation comparing digestion profiles obtained after snake venom phosphodiesterase digestion of (FIG. 5A) oligonucleotide analog (SEQ ID NO:4) and (FIG. 5B) control oligonucleotide (SEQ ID NO:5);
FIG. 6 is an autoradiogram showing the phosphodiesterase I digestion pattern of oligonucleotides 1-3 (SEQ ID NOS:1-3) and d(A)₁₁ (SEQ ID NO:6) at different time intervals; and
FIG. 7 is an autoradiogram of a polyacrylamide gel demonstrating the stability of oligonucleotides having SEQ ID NO:4 and SEQ ID NO:5 in the presence of 10% fetal bovine serum.

### DETAILED DESCRIPTION OF THE PREFERRED EMBODIMENTS

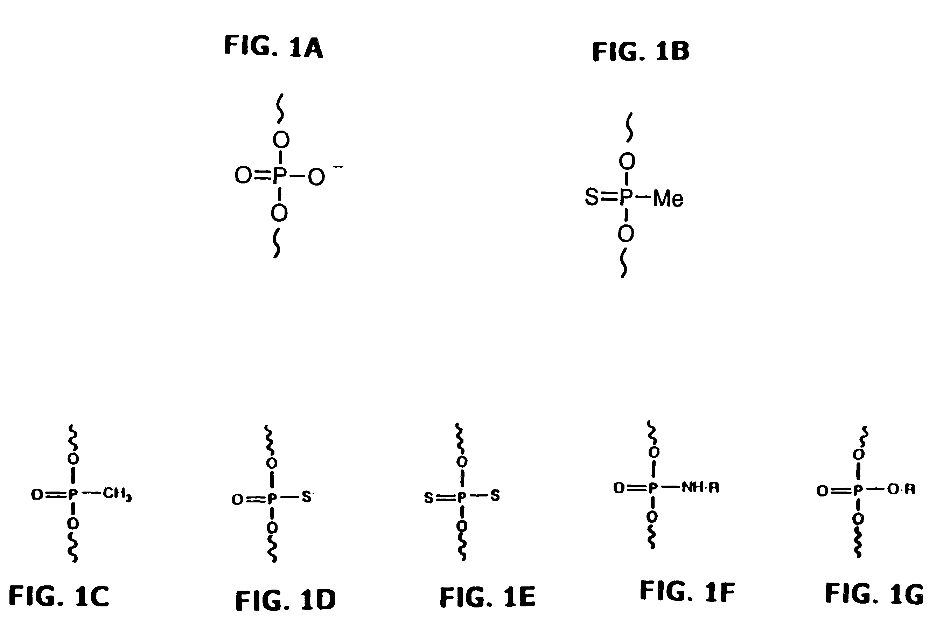

The present invention provides oligonucleotide analogs characterized by having at least one internucleotide linkage that is an alkylphosphonate or alkylphosphonothioate linkage. The remaining linkages can be phosphodiester linkages or other artificial internucleotide linkages including but not limited to phosphorothioates, phosphorodithioates, phosphoramidates, and phosphate esters. Some of these linkages are depicted in FIG. 1. Such modification of the nucleic acid sugar backbone improves cellular uptake and resistance to nuclease action. The number of alkylphosphonate or alkylphosphonothioate linkages can range from 1 to as many internucleotide linkages as are present in the oligonucleotide. Thus, for purposes of the invention, the term "oligonucleotide alkylphosphonate" or "oligonucleotide alkylphosphonothioate" is intended to encompass every such embodiment.

In a preferred embodiment, oligonucleotides according to the invention will range from about 2 to about 60 nucleotides in length, and most preferably from about 25 to about 30 nucleotides in length. Accordingly, in these embodiments, oligonucleotides of the invention will have from 1 to about 59 and from 24 to 29 alkylphosphonate or alkylphosphonothioate internucleotide linkages, respectively.

In some embodiments of the invention, the oligonucleotides have at least one, and preferably 6 to 10 2'-substituted ribonucleoside. Useful 2'-substitutions include, *e.g.,* 2'-O-methyl, 2'-allyl, 2'-aryl, 2'-alkyl, 2'-halo, or 2'-amino, but not with 2'-H, wherein allyl, aryl, or alkyl groups may be unsubstituted or substituted, *e.g.,* with halo, hydroxy, trifluoromethyl, cyano, nitro, acyl, acyloxy, alkoxy, carboxyl, carbalkoxyl or amino groups. These substitutions can be performed by methods well known to the skilled artisan (see, *e.g.,* Goodchild (1990) *Bioconjugate Chem.* 1:165-187). In a particularly useful oligonucleotide, there are 6 or more ribonucleosides and/or 2'-substituted ribonucleosides to enhance duplex stability.

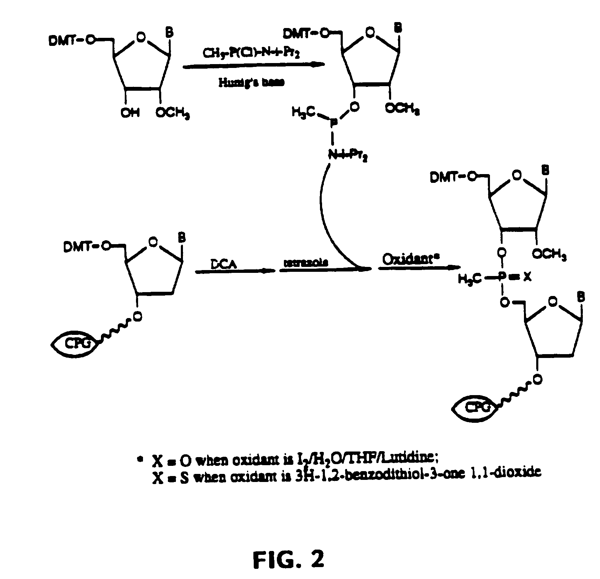

The oligonucleotide alkylphosphonate analogs of the invention can be prepared, for example, by synthesizing 3'-phosphonamidites of 2'-O-Me 5'-DMT ribonucleosides essentially according to the method of Agrawal and Goodchild (*Tetr.Lett.*(*1987*) 28:3539-3542). This procedure is diagrammed in FIG. 2. The dry phosphonamidites thus produced may then be used on an automated DNA synthesizer to make the required oligonucleotides. Coupling is carried out using suitably protected 2'-O-methyl-ribonucleoside methylphosphonamidites at desired positions. The oligomers so synthesized are cleaved from the controlled pore (CPG) support and then deprotected with ethylenediamine. Representative oligonucleotide analogs of the invention are set forth as SEQ ID NOS:1-4 and 7.

The oligonucleotide methylphosphonothioates of the invention can be prepared by the same methodology used for the synthesis of alkylphosphonates as shown in FIG. 2B except that the oxidant is a 1% solution of 3H-1,2-benzodithiol-3-one 1,1 dioxide (Iyer et al (1990) *J. Org. Chem.* 55:4693-4698) instead of I₂/H₂O/THF/lutidine.

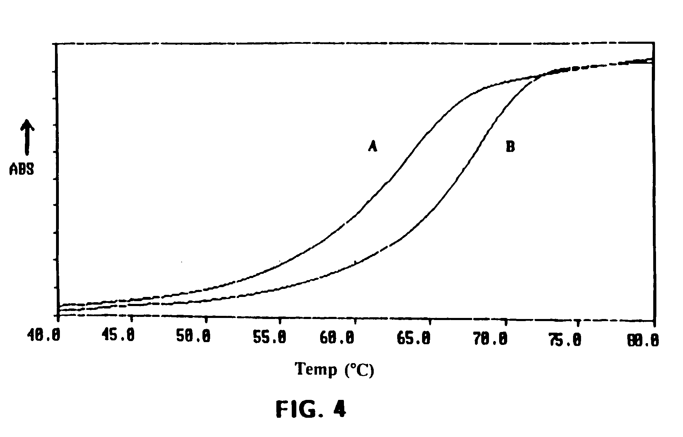

The oligonucleotide analogs so produced may then be tested for their ability to hybridize stably with complementary DNA and RNA strands by determining the temperature at which the analog-complementary strand duplexes melted. The melting curves of a representative oligonucleotide methylphosphonate analog which is 25 bases long (a 25mer) duplexed to (A) complementary DNA or to (B) complementary RNA are shown in FIG. 4. The melting temperature (Tm) values obtained with the modified 25mer (SEQ ID NO:4) 4 and the normal 25mer (SEQ ID NO:5) are summarized in TABLE 1. These values are the average of two experiments.

**TABLE 1**

| | Thermal Melting Data | | |
|---|---|---|---|
| Oligomer | Complementary Strand | Tm, °C | % Hyper-Chromicity |
| 25mer analog | DNA | 63.7 | 19.2 |
| (SEQ ID NO:4) | RNA | 68.7 | 15.0 |
| | | | |
| 25mer analog | DNA | 65.7 | 22.3 |
| (SEQ ID NO:5) | RNA | 71.8 | 18.1 |

These results suggest that the methylphosphate modification does not hinder the ability of the oligonucleotide to form a duplex with a complementary strand of DNA or RNA, nor does its presence destabilize duplex formation.

To show that the methylphosphonate linkage is incorporated into the oligonucleotide and that the presence of this modification does not prevent subsequent addition and extension of the nucleotide chain, representative oligonucleotide analogs 1-3 (SEQ ID NOS:1-3) with one, two, and three methyl-phosphonate linkages, respectively, were synthesized and studied by ion exchange HPLC. As expected, the unmodified d(A)₁₁ control was eluated at 30 minutes on the PARTISIL SAX column. Oligonucleotide analogs 1-3 (SEQ ID NO:1-3) were eluted at 23, 20, and 18 minutes, respectively, using a 0 to 50% gradient of solvent A (60% formamide, 40% water, 1 mM potassium dihydrogen phosphonate, pH 6.3) and solvent B (60% formamide, 40% water, 300 mM potassium dihydrogen phosphate, pH 6.3).

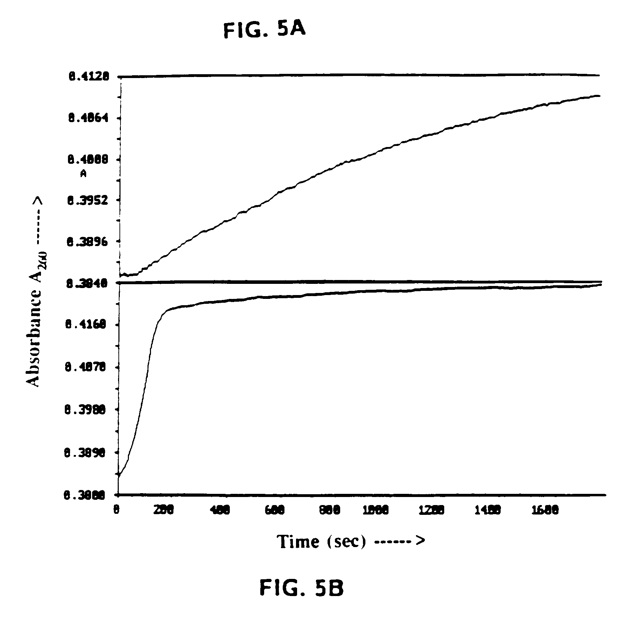

The stability of oligonucleotide methylphosphonate analogs was examined in the presence of snake venom phosphodiesterase (SVPD) or phosphodiesterase I, a 3'-exonuclease using the 25mer (SEQ ID NO:4) as the sample. Increase in absorbance due to the hydrolysis of an oligonucleotide was monitored on a spectrometer. The resulting digestion profiles of the 25mer analog (SEQ ID NO:4) and the control 25mer (SEQ ID NO:5) are shown in FIG. 5. 50% of the control 25mer (SEQ ID NO:5) was digested in about 100 seconds. However, the half life of the modified 25mer (SEQ ID NO:4) was measured as 800-900 seconds under the same experimental conditions indicating that the modification is resistent to nuclease action.

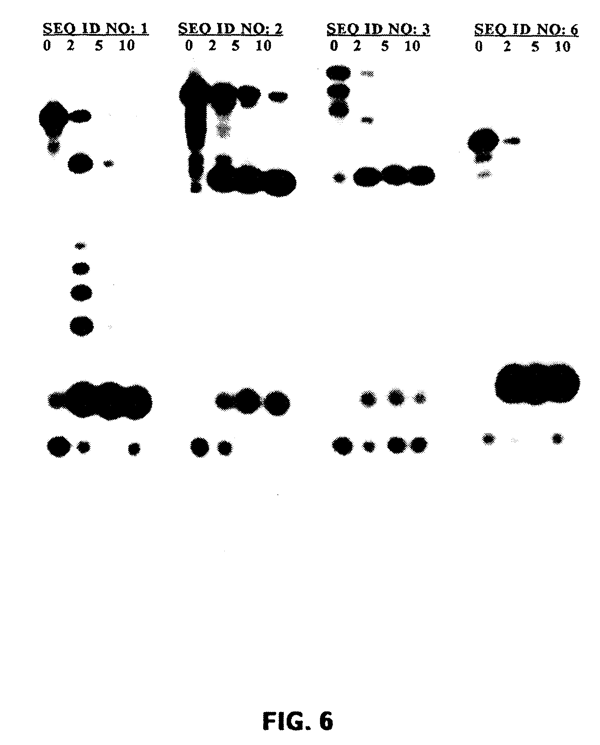

The stability of the oligonucleotides was also examined in the presence of phosphodiesterase I. The d(A)₁₁ (SEQ ID NO:6) oligonucleotide was used as the control. The results are shown in FIG. 6. The control (SEQ ID NO:6) was completely degraded into mononucleotides within 5 minutes. The oligonucleotide 1 (SEQ ID NO:1) which contains one methylphosphonate linkage, is slightly more stable than d(A)₁₁ (SEQ ID NO:6). However, in 10 minutes, almost all of oligonucleotide 1 (SEQ ID NO:6) was digested. In the case of oligonucleotides 2 and 3 (SEQ ID NOS: 2 and 3) which contain two and three methylphosphonate linkages, respectively, degradation stopped at nucleotide 6 or 7. This suggests that the phosphodiesterase I may cleave the first methylphosphonate from the 3'-end but cannot cleave the second one.

In another aspect, the invention provides oligonucleotide analogs that are effective in inhibiting viruses, pathogenic organisms, or the expression of cellular genes. The ability to inhibit such agents is clearly important to the treatment of a variety of disease states. Oligonucleotide analogs according to this aspect of the invention share the characteristics of the above-described oligonucleotide analogs, and also have a nucleotide sequence that is complementary to a nucleic acid sequence that is from a virus, a pathogenic organism, or a cellular gene. The oligonucleotide analogs are from 2 to 60, preferably 8 to 60, more preferably 25 to 30 nucleotides in length. For purposes of the invention, the term "oligonucleotide sequence that is complementary to a nucleic acid sequence" is intended to mean an oligonucleotide sequence that hybridizes to the nucleic acid sequence under physiological conditions, *e.g.,* by Watson-Crick base pairing (interaction between oligonucleotide and single-stranded nucleic acid), by Hoogsteen base pairing (interaction between oligonucleotide and double-stranded nucleic acid), or by any other means. Such hybridization under physiological conditions is measured as a practical matter by observing interference with the function of the nucleic acid sequence.

The nucleic acid sequence to which an oligoribonucleotide analog of the invention is complementary will vary, depending upon the agent to be inhibited or the nucleic acid to be targeted. In many cases the nucleic acid sequence will be a viral nucleic acid sequence. The use of antisense oligonucleotides to inhibit various viruses is well known, and has recently been reviewed in Agrawal (*Tibtech* (1992) 10:152-15). Viral nucleic acid sequences that are complementary to effective antisense oligonucleotides have been described for many viruses, including human immunodeficiency virus type 1 (U.S. Patent No. 4,806,463), *Herpes simplex* Virus (U.S. Patent No. 4,689,320), Influenza virus (U.S. Patent No. 5,194,428), and Human papilloma virus (Storey at al., (1991) *Nucleic Acids Res.* 19:4109-4114). Oligonucleotides according to the invention can have sequences complementary to any of these viral nucleic acid sequences. Additional viruses that have known nucleic acid sequences against which antisense oligonucleotides can be prepared include Foot and Mouth Disease Virus (*See* Robertson et al., (1985) *J. Virol.* 54:651; Harris et al., (1980) *J. Virol.* 36:659); Yellow Fever Virus (*See* Rice et al., (1985) *Science* 229:726), Varicella-Zoster Virus (*See* Davison and Scott, (1986) *J. Gen. Virol.* 67:2279) ; and Cucumber Mosaic Virus (*see* Richards at al,(1978) *Virol.* 89:395).

Alternatively, oligonucleotides according to the invention can have a nucleic acid sequence complementary to a nucleic acid sequence of a pathogenic organism. The nucleic acid sequences of many pathogenic prokaryotes have been described, and include the malaria organism, *Plasmodium falciparum,* and many other pathogenic bacteria. Examples of pathogenic eucaryotea having known nucleic acid sequences against which antisense oligonucleotides can be prepared include *Trypanosoma brucei gambiense* and *Leishmania* (*see* Campbell et al., (1984) *Nature* 311:350); *Fasciola hepatica* (*see* Zurita et al., (1987) *Proc.Natl.Acad.Sci.* USA 84:2340). Antifungal oligonucleotides can be prepared using a target hybridizing region having an oligonucleotide sequence that is complementary to a nucleic acid sequence from, *e.g.*, the chitin synthetase gene. Likewise, antibacterial oligonucleotides can be prepared using, *e.g.*, the alanine racemase gene.

The oligonucleotides according to the invention altematively can have an oligonucleotide sequence complementary to a cellular gene or gene transcript, the abnormal expression or product of which results in a disease state or disorder. The nucleic acid sequences of several such cellular genes have been described, including the prion protein (Stahl et al., (1991) *FASEB J.* 5:2799-2807), the amyloid-like protein associated with Alzheimer's disease (U.S. Patent No. 5,013,370), and various well-known oncogenes and proto-oncogenes, such as c*-myb,* c*-myc,* c*-abl,* and n*-ras.* In addition, oligonucleotides that inhibit the synthesis of structural proteins or enzymes involved largely or exclusively in spermatogenesis, sperm motility, the binding of the sperm to the egg or any other step affecting sperm viability may be used as contraceptives for men. Similarly, contraceptives for women may be oligoribonucleotides that inhibit the synthesis or function of proteins or enzymes involved in ovulation, fertilization, implantation, or in the biosynthesis of hormones involved in those processes.

The oligonucleotide analogs of the invention also can be used to control hypertension. Such analogs suppress the synthesis of angiotensin converting enzyme or related enzymes in the renin/angiotensin system. Similarly, platelet aggregation can be controlled by suppression of the synthesis of enzymes necessary for the synthesis of thromboxane A2 for use in myocardial and cerebral circulatory disorders, infarcts, arteriosclerosis, embolism and thrombosis. Deposition of cholesterol in arterial wall can be inhibited by suppression of the synthesis of fattyacyl co-enzyme A:cholesterol acyl transferase in arteriosclerosis. Inhibition of the synthesis of cholinephosphotransferase may be useful in hypolipidemia.

There are numerous neural disorders in which hybridization arrest can be used to reduce or eliminate adverse effects of the disorder. For example, suppression of the synthesis of monoamine oxidase can be used in Parkinson's disease; suppression of catechol-o-methyl transferase can be used to treat depression; and suppression of indole N-methyl transferase can be used in treating schizophrenia.

Suppression of selected enzymes in the arachidonic acid cascade which leads to prostaglandins and leukotrienes may be useful in the control of platelet aggregation, allergy, inflammation, pain and asthma.

Suppression of the protein expressed by the multidrug resistance (*mdr*) gene, which is responsible for development of resistance to a variety of anti-cancer drugs and is a major impediment in chemotherapy may prove to be beneficial in the treatment of cancer.

Oligonucleotide sequences complementary to nucleic acid sequences from any of these genes can be used for oligonucleotides according to the invention, as can be sequences complementary to any other cellular gene or gene transcript, the abnormal expression or product of which results in a disease state or disorder.

Antisense regulation of gene expression in plant cells has been described in U.S. Patent No. 5,107,065.

In another aspect, the invention provides a method for inhibiting the gene expression of a viral gene, a gene of a inhibiting the gene expression of a viral gene, a gene of a pathogenic organism, or a cellular gene using the oligonucleotide analogs described above. The method includes treating the cells infected with the virus or pathogenic organism in the former two cases, or the cells generally in the latter case, with the analogs of the invention in an amount sufficient to hybridize with available copies of the nucleic acid to which it is complementary. Treatment may be accomplished by directly administering the analogs to the cells, which may be cultured or in an organism, as described below.

In yet another aspect, the invention includes therapeutic pharmaceutical formulations that are effective for treating virus infection, infection by pathogenic organisms, or a disorder or disease resulting from abnormal gene expression or from the expression of an abnormal gene product. Such therapeutic pharmaceutical formulations contain the oligonucleotides according to the first aspect of the invention in a physiologically acceptable carrier.

As used herein, a "physiologically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

These pharmaceutical formulations can be used to practice yet another embodiment of the invention, namely, a method of treating a mammal having a disease or disorder resulting from infection with a virus or pathogenic organism, or from the abnormal expression or product of a cellular gene. The method comprises administering the therapeutic pharmaceutical formulation of the invention to the afflicted mammal in an amount sufficient to enable the binding of the oligonucleotide to the complementary nucleic acid to which it has been targeted. In this way, the oligonucleotide inhibits the expression and replication of the gene from the virus, pathogenic organism, or expression of the abnormal cellular gene.

Effective dosages of the oligonucleotide and modes of its administration in the treatment of various disorders such as AIDS can be determined by routine experimentation. The pharmaceutical forms suitable for injectable use include sterile aqueous solutions or dispersions and sterile powders for the extemporaneous preparation of sterile injectable solutions or dispersions. In all cases the form must be sterile. It must be stable under the conditions of manufacture and storage and may be preserved against the contaminating action of microorganisms, such as bacterial and fungi. The carrier can be a solvent or dispersion medium. The prevention of the action of microorganisms can be brought about by various antibacterial and antifungal agents. Prolonged absorption of the injectable therapeutic agents can be brought about by the use of the compositions of agents delaying absorption.

The oligonucleotide in the carrier may be administered by any useful route including intravenous or intraperitoneal injection, or by intranasal, rectal, oral, transdermal, or subcutaneous administration. Sterile injectable solutions are prepared by incorporating the oligonucleotide in the required amount in the appropriate solvent, followed by filtered sterilization.

The method of the invention may be used to treat variety of viral diseases including AIDS, ARC, oral or genital herpes, papilloma warts, flu, foot and mouth disease, yellow fever, chicken pox, shingles, HTLV-leukemia, and hepatitis. Among fungal diseases treatable by the method are candidiasis, histoplasmosis, cryptococcocis, blastomycosis, aspergillosis, sporotrichosis, chromomycosis, dermatophytosis and coccidioidomycosis. The method can also be used to treat rickettsial diseases (*e.g.,* typhus, Rocky Mountain spotted fever), as well as sexually transmitted diseases caused by *Chlamydia trachomatis* or *Lymphogranuloma venereum.* A variety of parasitic diseases can be treated by the method, including amebiasis, Chegas' disease, toxoplasmosis, pneumocystosis, giardiasis, cryptosporidiosis, trichomoniasis, and *Pneumocystis carini* pneumonia, worm (helminthic diseases) such as ascariasis, filariasis, trichinosis, schistosomiasis, and nematode or cestode infections. Malaria can be treated by the method regardless of whether it is caused by *P. falciparum, P.vivax. P.orale,* or *P. malariae.*

The infectious diseases identified above can all be treated by the method of the invention because the infectious agents for these diseases are known, and thus oligonucleotides according to the invention can be prepared to have an nucleic acid sequence that is complementary to a nucleic acid sequence essential for the propagation of the infectious agent (e.g., an essential gene).

Other disease states or conditions that are treatable by the method result from an abnormal expression or product of a cellular gene. These conditions have been discussed above.

The following example illustrates the preferred mode of making and practicing the present invention, but is not meant to limit the scope of the invention since alternative methods may be utilized to obtain similar results.

### EXAMPLES

### 1. Synthesis of Ribonucleoside Phosphonamidites

3'-phosphonamidites of 2'-O-methyl 5'-DMT ribonucleosides were synthesized as described by Agrawal and Goodchild (Agrawal et al. (1987) *Tetr.Lett.* 28:3539-3542). This procedure is shown in FIG. 2. Briefly, to 0.024 mmol of 2'-O-methyl 5'-DMT ribonucleoside is added 0.4 mmol of Hunig's base (ethyl diisopropyl amine) in 5 ml of anhydrous dichloromethane. This is cooled in an ice bath for 10 minutes. 0.4 mmol of methyl N, N-diisopropylchloro phosphoramidite is added slowly in 2 ml of dichloromethane. The temperature of the solution is allowed to rise to ambient level, after which time it is left at room temperature for 30 minutes. The reaction is then quenched by adding 30 ml of ethyl acetate. The mixture is extracted with saturated sodium bicarbonate and brine solutions. The organic layer is dried over anhydrous sodium sulfate and evaporated to dryness. The resulting phosphonamidites are dissolved in a small amount of ethyl acetate and then precipitated by adding the ethyl acetate solution to a large volume of pentane at-30°C. The precipitate is collected by centrifugation and dried.

### 2. Oligonucleotide Synthesis

Dry phosphoramidites are dissolved in DNA synthesis grade acetonitrile at a concentration of 30 mg/ml and are used on an automated DNA synthesizer to make the required oligonucleotides. The oligonucleotide synthesis is carried out on Milligen/Biosearch 8700 automatic synthesizer using RNA synthesis cycle at 1 µM scale using the phosphoramidite approach (Agrawal et al., (1987) *Tetra Lett.* **28**:3539-3542). Coupling is carried out using suitably protected 2'-O-methylribonucleoside methylphosphonamidites at required positions for oligonucleotide analogs 1-4 (SEQ ID NOS:1-4).

### 3. Deprotection

The oligomers are cleaved from CPG using 1:1 ammonium hydroxide-ethanol mixture for I hour at room temperature and then deprotected with ethylenediamine at room temperature for 6 hours.

### 4. Purification and Assay

The 5'-DMT protected oligomers are purified by reverse phase chromatography using reverse phase (RP) C18 HPLC column using o.1M ammonium acetate and a 1:4 ratio of 0.1M ammonium acetate-acetonitrile gradient from 0 to 50%. The oligomers are detritylated with 80% acetic acid at room temperature for 1 hour and then desalted on a C₁₈ SEP-PACK cartridge (Agrawal et al., (1987) *Tetra. Lett.* **28**:3539-3542). The oligomers are then purified on 20% polyacrylamide denaturing gel. The oligomers are again desalted on SEP-PACK cartridge and used for further studies.

To show that the methylphosphonate linkage is incorporated into the oligonucleotide and that the presence of this modification does not prevent subsequent addition and extension of the nucleotide chain, analogs 1-3 (SEQ ID NOS:1-3) were assayed by ion exchange HPLC. A 0 to 50% gradient of solvents A (60% formamide, 40% water, 1 mM potassium dihydrogen phosphate, pH 6.3) and B (60% formamide, 40% water, 300 mM potassium dihydrogephosphate, pH 6.3) was used.

### 5. Hybridization Studies

Hybridization properties of the modified 25mer (SEQ ID NO:4) were studied by performing thermal melting experiments with DNA and RNA complementary strands. Typically, the modified oligonucleotide and the complementary strand (1:1) were dried in an Eppendorf tube and dissolved in 1 ml of 100 mM NaCl and 10 mM phosphate buffer, pH 7.4. The solution is heated to 80°C for 5 minutes and allowed to come to room temperature slowly. The Tm is measured on Perkin Elmer Lambda 2 UV/Vis Spectrometer interfaced with a Digital DECstation 316sx computer. The resulting melting curves of the 25mer analog with a complementary strand of A(DNA) and B(RNA) are shown in FIG. 4. The melting temperature (Tm) values obtained with the modified 25mer (SEQ ID NO:4) and the normal control 25mer (SEQ ID NO:5) are summarized in TABLE I. These values are the average of two experimments.

### 6. Stability of Oligonucleotide Analogs against Nucleases

The stability of the modified 25mer (SEQ ID NO:4) was examined in the presence of snake venom phosphodiesterase (SVPD), or phosphodiesterase I, a 3'-exonuclease. Increase in absorbance due to the hydrolysis of an oligonucleotide was monitored on a Perkin Elmer Lambda 2 UV/Vis Spectrometer. In a typical experiment, 0.2 A₂₆₀ units of modified oligonucleotide was dissolved in 0.5 ml of 10 mM MgCl₂, 10 mM Tris buffer, pH 8.3, and equilibrated at 37°C for 5 minutes. 1.5 x 10⁻³ units of SVPD was added, and the rate of digestion followed by recording change in absorbance at 260 nm with time. The resulting digestion profiled are shown in FIG. 5.

The stability of the oligonucleotides 1-3 (SEQ ID NOS:1-3) with reference to the control normal oligonucleotide d(A)₁₁ (SEQ ID NO:6) was studied with 3'-phosphodiesterase I by polyacrylamide gel electrophoresis. The oligoribonucleotides (about 100 ng) were labelled at their 5'end with ³P using τ-³P-ATP and T4 polynucleotide kinase (Sambrook et al., *Molecular Cloning, A Laboratory Manual* (2d ed.) Cold Spring Harbor Laboratory Press, 1989). Each 5'-end labelled oligoribonucleotide (3 µg) was treated with 1µl if phosphodiesterase I (1.5 x 10⁻⁴ Units) in 30 µl of buffer containing 50 mM Tris-HCl, pH 7.5, 5 mM MgCl₂, 5 mM DTT, 50 µg/ml BSA. 4 µl aliquot of sample was removed from the reaction mixture at 0, 2, 5, and 10 minutes intervals. The aliquots were heated at 90°C for 5 minutes and separated by electrophoresis on a 20% polyacrylamide-8M urea gel. The autoradiogram developed is shown in FIG. 6.

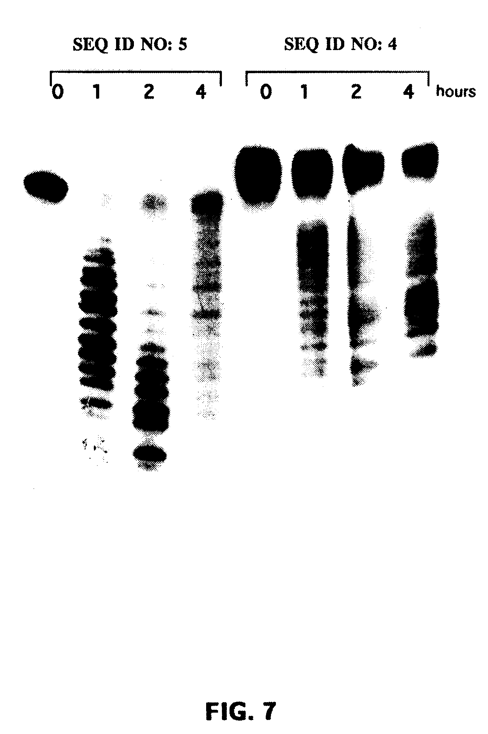

The stability of the modified 25mer analog (SEQ ID NO:4) with reference to the control 25mer oligonucleotide (SEQ ID NO:5) was studied in cell culture media containing 10% fetal bovine serum. The oligonucleotides (about 100 ng) were labelled at the 5'-end with ³P as described in the preceding paragraph. About 10 ng of each of the labelled oligonucleotides (specific activity 10⁶ dpm/10 ng), were separately incubated in 300 µl of SMEM cell culture media (GIBCO) containing 10% fetal bovine serum. At each time point (0, 1, 2, and 4 hr), 50 µl aliquots were removed, treated with 10 µl of proteinase K, phenol-chloroform extracted, and then precipitated with ethanol. The resulting reaction mixtures were analyzed by electrophoresis on a 20% polyacrylamide gel containing 8M urea. The developed autoradiogram is shown in FIG. 7.

The results show that the modified 25mer oligonucleotide analog is intact for 4 hours in considerable amounts compared to the control 25mer which is degraded into shorter sequences in less than 1 hour.

### 7. Inhibition of HIV

Oligonucleotide methylphosphonates are tested for their ability to inhibit HIV-1 in tissue culture as follows. H9 lymphocytes are infected with HIV-1 virions (≡0.01 - 0.1 TCID₅₀/cell) for one hour at 37°C. After one hour, unadsorbed virions are washed and the infected cells are divided among wells of 24-well plates. To the infected cells, an appropriate concentration (from stock solution) of oligonucleotide analog is added to obtain the required concentration in 2 ml medium. The cells are then cultured for four days. At the end of four days, level of HIV-1 expression is measured by synthetic formation, p24 expression, and reverse transcriptase activity. The level of expression of p24 in infected cells treated with oligonucleotide analog is compared with the level detected in untreated infected cells.

## Claims

1. An oligonucleotide analog comprising from 2 to 60 nucleotides, wherein at least one of the nucleotides is a ribonucleotide alkylphosphonate or alkylphosphonothioate and at least one of the nucleotides is a deoxyribonucleotide.

2. The oligonucleotide analog of claim 1 having from 8 to 60 nucleotides.

3. The oligonucleotide analog of claim 2 having from 25 to 30 nucleotides.

4. The oligonucleotide analog of claim 2 having from 2 to 60 akylphosphonates or alkylphosphonothioates.

5. The oligonucleotide analog of claim 4 having from 25 to 30 alkylphosphonates or alkylphosphonothioates.

6. The oligonucleotide analog of claim 2 having from 7 to 10 alkylphosphonothioates.

7. The oligonucleotide analog of claim 1 wherein at least one ribonucleotide is substituted at the 2' position of the ribose group with one of the group consisting of 2'-O-methyl, 2'-allyl, 2'-aryl, 2'-alkyl, 2'-halo, and 2'-amino.

8. The oligonucleotide analog of claim 1 wherein at least one nucleotide is a 2'-substituted ribonucleotide.

9. The oligonucleotide analog of claim 8 wherein the alkyl moiety of the alkylphosphonate and alkylphosphonothioate is chosen from the group consisting of methyl, ethyl, propyl, and butyl.

10. The oligonucleotide analog of claim 9 wherein the 2'-substituted ribonucleotide is further substituted at its 2'-allyl, 2'-aryl or 2'-alkyl with one of the group consisting of halo. hydroxy, cyano, nitro, acyl, acyloxy, alkoxy, carboxyl, trifluoromethyl, carbalkoxyl, and amino groups.

11. The oligonucleotide analog of claim 1 comprising a nucleotide sequence that is complementary to a nucleic acid sequence from a virus, pathogenic organism, or cellular gene or cellular gene transcript, the abnormal expression of which results in a disease state.

12. A therapeutic pharmaceutical formulation comprising:
(a) the oligonucleotide of anyone of claims 1 to 11; and
(b) a physiologically acceptable carrier.

13. A method of inhibiting the expression of a gene in vitro from a virus , pathogenic organism, or cell, the expression of which is associated with a disease state, the method comprising the step of:
treating the virus, pathogenic organism or cell with the oligonucleotide of anyone of claims 1 to 11 that is complementary to a nucleic acid sequence from the virus, pathogenic organism, or cellular gene or cellular gene transcript.

14. An oligonucleotide according to anyone of claims 1 to 11 for use as an active therapeutic substance.

15. The use of an oligonucleotide according to anyone of claims 1 to 11 for the preparation of a pharmaceutical formulation for inhibiting the expression of a gene from a virus, pathogenic organism, or cell.

## Patentansprüche

1. Ein Oligonucleotid umfassend 2 bis 60 Nucleotide, in dem mindestens eines der Nucleotide ein Alkylphosphonat- oder Alkylphosphonothioatribonucleotid und mindestens eines der Nucleotide ein Desoxyribonucleotid ist.

2. Das Oligonucleotid nach Anspruch 1 mit 8 bis 60 Nucleotiden.

3. Das Oligonucleotid nach Anspruch 2 mit 25 bis 30 Nucleotiden.

4. Das Oligonucleotid nach Anspruch 2 mit 2 bis 60 Alkylphosphonaten oder Alkylphosphonothioaten.

5. Das Oligonucleotid nach Anspruch 4 mit 25 bis 30 Alkylphosponaten oder Alkylphosphonothioaten.

6. Das Oligonucleotid nach Anspruch 2 mit 7 bis 10 Alkylphosphonothioaten.

7. Das Oligonucleotid nach Anspruch 1, in dem mindestens ein Ribonucleotid an der 2'-Position des Riboserestes mit einem Rest bestehend aus 2'-O-Methyl, 2'-Allyl, 2'-Aryl, 2'-Alkyl, 2'-Halo und 2'-Amino substituiert ist.

8. Das Oligonucleotid nach Anspruch 1, in dem mindestens ein Nucleotid ein 2'-substituiertes Ribonucleotid ist.

9. Das Oligonucleotid nach Anspruch 8, in dem der Alkylteil des Alkylphosphonats und Alkylphosphonothioats aus den Resten Methyl, Ethyl, Propyl und Butyl ausgewählt ist.

10. Das Oligonucleotid nach Anspruch 9, in dem das 2'-substituierte Ribonucleotid zusätzlich an seinem 2'-Allyl, 2'-Aryl oder 2'-Alkyl mit einem Rest bestehend aus Halo, Hydroxy, Cyano, Nitro, Acyl, Acyloxy, Alkoxy, Carboxyl, Trifluoromethyl, Carbalkoxyl und Amino substituiert ist.

11. Das Oligonucleotid nach Anspruch 1 umfassend eine Nucleotidsequenz, die komplementär zu einer Nucleinsäuresequenz eines Virus, eines pathogenen Organismus, oder eines zellulären Gens oder zellulären Gentranskripts ist, dessen abnormale Expression zu einem Krankheitszustand führt.

12. Eine therapeutische pharmazeutische Formulierung umfassend:
(a) ein Oligonucleotid nach einem der Ansprüche 1 bis 11; und
(b) einen physiologisch annehmbaren Träger.

13. Eine Methode zur Hemmung der Expression eines Gens *in vitro* von einem Virus, einem pathogenen Organismus, oder einer Zelle, dessen Expression mit einem Krankheitszustand assoziiert ist, wobei die Methode die Schritte umfaßt:
Behandeln des Virus, des pathogenen Organismus oder der Zelle mit dem Oligonucleotid nach einem der Ansprüche 1 bis 11, das komplementär zu einer Nucleinsäuresequenz eines Virus, eines pathogenen Organismus, oder eines zellulären Gens oder zellulären Gentranskripts ist.

14. Ein Oligonucleotid nach einem der Ansprüche 1 bis 11 zur Verwendung als eine aktive therapeutische Substanz.

15. Die Verwendung eines Oligonucleotids nach einem der Anpsrüche 1 bis 11 zur Herstellung einer pharmazeutischen Formulierung zur Hemmung der Expression eines Gens von einem Virus, einem pathogenen Organismus oder einer Zelle.

## Revendications

1. Analogue oligonucléotidique comprenant de 2 à 60 nucléotides, dans lequel au moins un des nucléotides est un ribonucléotide alcoylphosphonate ou alcoylphosphonothioate et au moins un des nucléotides est un désoxyribonucléotide.

2. Analogue oligonucléotidique selon la revendication 1, constitué de 8 à 60 nucléotides.

3. Analogue oligonucléotidique selon la revendication 2, constitué de 25 à 30 nucléotides.

4. Analogue oligonucléotidique selon la revendication 2, comportant de 2 à 60 alcoylphosphonates ou alcoylphosphonothioates.

5. Analogue oligonucléotidique selon la revendication 4, comportant de 25 à 30 alcoylphosphonates ou alcoylphosphonothioates.

6. Analogue oligonucléotidique selon la revendication 2, comportant de 7 à 10 alcoylphosphonothioates.

7. Analogue oligonucléotidique selon la revendication 1, dans lequel au moins un ribonucléotide est substitué dans la position 2' du groupe ribose par un membre du groupe constitué par 2'-O-méthyle, 2'-allyle, 2'-aryle, 2'-alcoyle, 2'-halo et 2'-amino.

8. Analogue oligonucléotidique selon la revendication 1, dans lequel au moins un nucléotide est un ribonucléotide substitué en position 2'.

9. Analogue oligonucléotidique selon la revendication 8, dans lequel la partie alcoyle de l'alcoylphosphonate ou de l'alcoylphosphonothioate est choisi dans le groupe constitué par les groupes méthyle, éthyle, propyle et butyle.

10. Analogue oligonucléotidique selon la revendication 9, dans lequel le ribonucléotide substitué en position 2' est en outre substitué sur sa partie 2'-allyle, 2'-aryle, ou 2'-alcoyle par un membre du groupe constitué par les groupes halo, hydroxy, cyano, nitro, acyle, acyloxy, alkoxy, carboxyle, trifluorométhyle, carbalkoxyle et amino.

11. Analogue oligonucléotidique selon la revendication 1, comprenant une séquence nucléotidique qui est complémentaire d'une séquence d'acide nucléique d'un gène d'un virus, d'un organisme pathogène ou d'une cellule, ou d'un transcrit d'un gène cellulaire, dont l'expression anormale provoque un état pathologique.

12. Formulation pharmaceutique thérapeutique comprenant :
(a) l'oligonucléotide selon l'une quelconque des revendications 1 à 11 ; et
(b) un véhicule acceptable au plan physiologique.

13. Procédé d'inhibition *in vitro* de l'expression d'un gène d'un virus, d'un organisme pathogène, ou d'une cellule dont l'expression est associée à un état pathologique, le procédé comprenant les étapes consistant à :
traiter le virus, l'organisme pathogène ou la cellule par l'oligonucléotide selon l'une quelconque des revendications 1 à 11, qui est complémentaire d'une séquence d'acide nucléique du gène du virus, de l'organisme pathogène ou de la cellule ou du transcrit du gène de la cellule.

14. Oligonucléotide selon l'une quelconque des revendication 1 à 11, destiné à être utilisé en tant que substance active thérapeutique.

15. Utilisation d'un oligonucléotide selon l'une quelconque des revendications 1 à 11, pour la préparation d'une formulation pharmaceutique destinée à inhiber l'expression d'un gène de virus, d'un organisme pathogène ou d'une cellule.


### SEQUENCE LISTING

(1) GENERAL INFORMATION:
   (i) APPLICANTS: HYBRIDON, INC.
   (ii) TITLE OF INVENTION: Oligonucleotide Alkylphosphonates and Alkylphosphonothioates
   (iii) NUMBER OF SEQUENCES: 7
   (iv) CORRESPONDENCE ADDRESS:
      (A) ADDRESSEE: Allegretti & Witcoff, Ltd.
      (B) STREET: 10 S. Wacker Drive, Suite 3000
      (C) CITY: Chicago
      (D) STATE: Illinois
      (E) COUNTRY: U.S.A.
      (F) ZIP: 60606
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US/08/009,262
      (B) FILING DATE: 01/25/93
      (C) CLASSIFICATION:
   (viii) ATTORNEY/AGENT INFORMATION:
      (A) NAME: Greenfield, Michael S.
      (B) REGISTRATION NUMBER: 37,142
      (C) REFERENCE/DOCKET NUMBER: 92,774-A
   (ix) TELECOMMUNICATION INFORMATION:
      (A) TELEPHONE: 312/715-1000
      (B) TELEFAX: 312/715-1234
(2) INFORMATION FOR SEQ ID NO:1:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: mRNA
   (iii) HYPOTHETICAL: NO
   (iv) ANTI-SENSE: YES
   (ix) FEATURE:
      (A) NAME/KEY:
      (B) LOCATION:
      (D) OTHER INFORMATION: /product= "phosphodiester linkages between bases 6-7; U is 2-O-methyl"
   (xi) SEQUENCE DESCRIPTION: SEQ ID NO:1:
   TTTTTUTTTT T 11
(2) INFORMATION FOR SEQ ID NO:2:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: mRNA
   (iii) HYPOTHETICAL: NO
   (iv) ANTI-SENSE: YES
   (ix) FEATURE:
      (A) NAME/KEY:
      (B) LOCATION:
      (D) OTHER INFORMATION: /product= "phosphodiester linkages between nucleosides 3-4, 9-10; U is 2-O-methyl"
   (xi) SEQUENCE DESCRIPTION: SEQ ID NO:2:
   TTUTTTTTUT T 11
(2) INFORMATION FOR SEQ ID NO:3:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: mRNA
   (iii) HYPOTHETICAL: NO
   (iv) ANTI-SENSE: YES
   (ix) FEATURE:
      (A) NAME/KEY:
      (B) LOCATION:
      (D) OTHER INFORMATION: /product= "phosphodiester linkages between nucleosides 3-4, 6-7, 9-10; U is 2-O-methyl"
   (xi) SEQUENCE DESCRIPTION: SEQ ID NO:3:
   TTUTTUTTUT T 11
(2) INFORMATION FOR SEQ ID NO:4:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: mRNA
   (iii) HYPOTHETICAL: NO
   (iv) ANTI-SENSE: YES
   (ix) FEATURE:
      (A) NAME/KEY:
      (B) LOCATION:
      (D) OTHER INFORMATION: /product= "phosphodiester linkages between nucleosides 21-22, 22-23, 23-24, and 24-25; bases 21-24 are 2-O-methyl"
   (xi) SEQUENCE DESCRIPTION: SEQ ID NO:4:
   CTCTCGCACC CATCTCTCTC CUUCT 25
(2) INFORMATION FOR SEQ ID NO:5:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: mRNA
   (iii) HYPOTHETICAL: NO
   (iv) ANTI-SENSE: YES
   (xi) SEQUENCE DESCRIPTION: SEQ ID NO:5:
   CTCTCGCACC CATCTCTCTC CTTCT 25
(2) INFORMATION FOR SEQ ID NO:6:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: mRNA
   (iii) HYPOTHETICAL: NO
   (iv) ANTI-SENSE: YES
   (xi) SEQUENCE DESCRIPTION: SEQ ID NO:6
   AAAAAAAAAA A 11
(2) INFORMATION FOR SEQ ID NO:7:
   (i) SEQUENCE CHARACTERISTICS:
      (A) LENGTH: 8 base pairs
      (B) TYPE: nucleic acid
      (C) STRANDEDNESS: single
      (D) TOPOLOGY: linear
   (ii) MOLECULE TYPE: mRNA
   (iii) HYPOTHETICAL: NO
   (iv) ANTI-SENSE: YES
   (ix) FEATURE:
      (A) NAME/KEY:
      (B) LOCATION:
      (D) OTHER INFORMATION: /product= "phosphodiester linkages between nucleosides 7-8; C and U are 2-0-methyl"
   (xi) SEQUENCE DESCRIPTION: SEQ ID NO:7:
   CCUUCCCT 8